# EUROPEAN PATENT APPLICATION

(11) **EP 4 438 617 A1**
(43) Date of publication of application: **02.10.2024**
(21) Application number: 22899049.5
(22) Date of filing: 24.11.2022
(51) Int. Cl.: C07K 7/08, A61K 38/00, A61P 25/28

(54) **PEPTIDE HAVING INHIBITORY ACTIVITY AGAINST AMYLOID PRECURSOR PROTEIN AND USE THEREOF**

(30) Priority: 24.11.2021 KR 20210163629; 24.11.2021 KR 20210163630
(71) Applicant: Wingstabio Inc., Hanam-si, Gyeonggi-do 12902 (KR)
(72) Inventor: LEE, Joong Chul, Yongin-si, Gyeonggi-do 16903 (KR)
(74) Representative: BCKIP Part mbB
(86) International application number: PCT/KR2022/018703
(87) International publication number: WO 2023/096367

(57) **Abstract**

The present application relates to a peptide having inhibitory activity against amyloid precursor protein and a use thereof and provides a peptide consisting of amino acid sequence of any one of SEQ ID NO: 1 to SEQ ID NO: 8 and SEQ ID NO: 20 to SEQ ID NO: 23 and a pharmaceutical composition for preventing or treating a neurodegenerative disease, including the peptide as an active ingredient.

## Description

### [Technical Field]

The present application relates to a peptide having inhibitory activity against amyloid precursor protein and use thereof. The present patent application claims priority to Korean Patent Application No. 10-2021-0163629 submitted to the Korean Intellectual Property Office on November 24, 2021, and Korean Patent Application No. 10-2021-0163630 submitted to the Korean Intellectual Property Office on November 24, 2021, and the disclosure of the above patent applications is incorporated herein by reference.

### [Background Art]

Alzheimer's disease (AD) is a disease that exhibits symptoms of gradual memory decline and cognitive loss, and its incidence rate is significantly increasing as the average human lifespan increases. One of the pathological characteristics of AD is senile plaques that accumulate on the outside of neurons, and one of the causative materials thereof is beta-amyloid (Aβ).

Aβ is a protein fragment cleaved from amyloid precursor protein (APP), and the most important enzyme involved in Aβ production is beta-secretase (β-secretase), which is named beta-site APP-cleaving enzyme (BACE1).

APP cleaved by BACE1 is divided into an approximate 90 kDa N-terminal domain called sAPPβ and a cytoplasmic domain called CTFβ (C99). The sAPPβ produced in this way is secreted out of a cell, and C99 is further cleaved by gamma-secretase (γ-secretase) to produce 4 kDa beta-amyloid.

Due to abnormal metabolism of APP, a large amount of Aβ is produced, causing brain cell toxicity and causing AD to develop. Therefore, it may be expected that a substance inhibiting the activity of APP may prevent or treat AD since it inhibits Aβ production.

Most of the drugs currently on the market or under development are drugs that improve the quality of life of AD patients by improving their symptoms, and examples of the drugs include tacrine, donepezil, rivastigmine, and galantamine, which are acetylcholinesterase inhibitors developed based on the cholinergic nervous system hypothesis, and memantine, which has an N-methyl-D-aspartate (NMDA)-glutamate receptor inhibitory function. However, since most of these therapeutic agents only provide a temporary improvement effect on clinical symptoms in the early stage of the disease and also cause side effects, treatment is difficult (Korean Patent No. 10-2085358).

The present inventors performed research to develop a therapeutic agent that can inhibit the production of Aβ protein or degrade it with the goal of fundamental treatment based on the cause of AD onset, and as a result, they developed a material that inhibits the expression and/or activity of APP and completed the present invention based on this.

### [Disclosure]

### [Technical Problem]

One aspect is to provide a peptide consisting of amino acid sequence of any one of SEQ ID NO: 1 to SEQ ID NO: 8 and SEQ ID NO: 20 to SEQ ID NO: 23.

Another aspect is to provide a pharmaceutical composition for preventing or treating a neurodegenerative disease including the peptide as an active ingredient.

Still another aspect is to provide a medicinal use of the peptide for preventing or treating a neurodegenerative disease, or a method of treating a neurodegenerative disease, including administering a therapeutically effective amount of the peptide to an individual.

Other objects and advantages of the present application will become clearer from the following detailed description together with the appended claims and drawings. Contents that are not described in the present specification may be fully recognized and inferred by a person skilled in the technical field of the present application or a similar technical field, so description thereof is omitted.

### [Technical Solution]

Each description and embodiment disclosed in the present application may also be applied to each other description and embodiment. In other words, all combinations of the various elements disclosed in the present application fall within the scope of the present application. In addition, the scope of the present application may not be considered as being limited by the specific description described below.

One aspect provides a peptide consisting of amino acid sequence of any one of SEQ ID NO: 1 to SEQ ID NO: 8 and SEQ ID NO: 20 to SEQ ID NO: 23.

The term "peptide," as used in the present specification, may refer to a linear molecule formed of linking amino acid residues to each other through peptide bonds. The peptide may be prepared according to a chemical synthesis method known in the art, especially solid-phase synthesis technology or liquid-phase synthesis technology (US Patent No. 5,516,891).

As a result of diligent efforts to develop a peptide having biologically effective activity, the present inventors identified a peptide consisting of amino acid sequence of any one of SEQ ID NO: 1 to SEQ ID NO: 8. Here, the biologically effective activity may represent any one or more selected from characteristics such as: (a) inhibition of amyloid precursor protein (APP) expression; (b) increased expression of a disintegrin and metalloproteinase-containing protein 10 (ADAM 10) protein or a disintegrin and metalloproteinase-containing protein 17 (ADAM 17) protein; (c) increased expression of insulin degrading enzyme (IDE) protein; (d) increased expression of anti-inflammatory cytokines; (e) promotion of neurite growth; and (f) increased expression of brain derived neurotrophic factor (BDNF).

Therefore, the peptide may be used for use in preventing or treating a neurodegenerative disease.

In the peptide, a protecting group may be attached to an N- or C- group of the peptide to acquire chemical stability, enhanced pharmacological properties (half-life, absorption, potency, efficacy, etc.), altered specificity (e.g., broad spectrum of biological activity), and reduced antigenicity. In one embodiment, an N-terminus of the peptide may be coupled to a protecting group of any one selected from the group consisting of an acetyl group, a fluoreonylmethoxycarbonyl group, a formyl group, a palmitoyl group, a myristyl group, a stearyl group, and polyethylene glycol (PEG) and/or a C-terminus of the peptide may be coupled to a protecting group of any one selected from the group consisting of an amino group (-NH₂) and azide (-NHNH₂). In addition, the peptide may optionally further include a targeting sequence, a tag, a labeled residue, and an amino acid sequence prepared for a specific purpose of increasing half-life or peptide stability.

The peptide may be artificially synthesized, or non-naturally occurring or engineered, and the term "non-naturally occurring or engineered" refers to a state created by applying artificial transformation rather than a state that exists in nature. Here, the artificial transformation may include artificially synthesizing an amino acid sequence by mimicking a plurality of amino acid structures or engineering it to acquire chemical stability, enhanced pharmacological properties, altered specificity, or reduced antigenicity as described above.

The term "stability," as used in the present specification, may refer to not only *in vivo* stability to protect the peptide from attack by protein cleavage enzymes in the body, but also storage stability (e.g., room temperature storage stability).

Another aspect provides a pharmaceutical composition for preventing or treating a neurodegenerative disease, including a peptide consisting of amino acid sequence of any one of SEQ ID NO: 1 to SEQ ID NO: 8 and SEQ ID NO: 20 to SEQ ID NO: 23 as an active ingredient.

Among the terms or elements mentioned in the description of the peptide, those that are the same as those already mentioned are as described above.

The term "prevention," as used in the present specification, refers to any action that inhibits or delays the onset of a disease by administering the composition.

The term "treatment," as used in the present specification, refers to any form of treatment provided to an individual suffering from a disease or at risk of developing a disease in order to provide an effect including improving the conditions(e.g., one or more symptoms) of the individual, delaying the progression of a disease, delaying the onset of symptoms, or slowing the progression of symptoms. Therefore, the terms "treatment" and "prevention" are not intended to mean cure or complete elimination of symptoms.

The "individual" refers to a subject in need of treatment for a disease, and more specifically, refers to mammals such as humans or non-human primates, mice, dogs, cats, horses, and cows.

The "neurodegenerative disease," which is a target disease to be prevented or treated by the pharmaceutical composition, may refer to a disease that causes various symptoms as degenerative changes occur due to gradual and steady death of neurons in the nervous system. The neurodegenerative disease may be selected from the group consisting of Alzheimer's disease (AD), Parkinson's disease, Huntington's disease, multiple sclerosis, mild cognitive impairment, cerebral amyloid angiopathy, amyloid stroke, systematic amyloidosis, amyloidosis-Dutch type, Niemann-Pick disease, senile dementia, amyotrophic lateral sclerosis, spinocerebellar atrophy (spinocerebellar ataxia), Tourette's syndrome, Friedreich's ataxia, Machado-Joseph's disease, Lewy body dementia, dystonia, progressive supranuclear palsy, frontotemporal dementia, peripheral neuropathy, and polyneuropathy, and it may preferably be AD, but is not limited thereto.

According to one embodiment, the peptide (Peptide-1 to Peptide-8) may inhibit the expression of APP, the main causative agent of a neurodegenerative disease such as AD, and at the same time, it may increase the expression of ADAM 10 and ADAM 17, which cleave previously produced APP on the surface of a cell membrane to release it in the form of secreted amyloid precursor protein alpha (sAPPa), and insulin-degrading enzyme (IDE), which degrades senile plaques that have already been produced. Furthermore, the peptide may alleviate a neuroinflammatory response, which is one of the main causes of a neurodegenerative disease, and regenerate a damaged nervous system.

According to one embodiment, the peptide (Peptide-9 to Peptide-12) may inhibit the expression of APP, the main causative agent of a neurodegenerative disease such as AD, and at the same time, it may increase the expression of ADAM 17, which cleaves previously produced APP on the surface of a cell membrane to release it in the form of sAPPa. Furthermore, the peptide may alleviate a neuroinflammatory response, which is one of the main causes of a neurodegenerative disease, and regenerate a damaged nervous system.

Therefore, the peptide may be used as an active ingredient in a pharmaceutical composition for preventing or treating a neurodegenerative disease.

The pharmaceutical composition may include a pharmaceutically effective amount of the peptide; and/or may include a pharmaceutically acceptable carrier, but is not limited thereto.

The term "pharmaceutically effective amount," as used in the present specification, refers to an amount sufficient to achieve the efficacy of the pharmaceutical composition in preventing or treating a neurodegenerative disease.

The pharmaceutically acceptable carrier is commonly used in formulation and includes lactose, dextrose, sucrose, sorbitol, mannitol, starch, gum acacia, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, and mineral oil, but is not limited thereto. Suitable pharmaceutically acceptable carriers and formulations are described in detail in Remington's Pharmaceutical Sciences (19th ed., 1995).

In addition to the above ingredients, the pharmaceutical composition may further include lubricants, wetting agents, sweeteners, flavoring agents, emulsifiers, suspending agents, and preservatives, but is not limited thereto.

The pharmaceutical composition may be administered orally or parenterally, preferably parenterally, and in case of parenteral administration, it can be administered by intramuscular injection, intravenous injection, subcutaneous injection, intraperitoneal injection, topical administration, transdermal administration or the like, but is not limited thereto.

The dosage of the pharmaceutical composition may be 0.0001 to 1000 µg (micrograms) 0.001 to 1000 µg, 0.01 to 1000 µg, 0.1 to 1000 µg, or 1.0 to 1000 µg per day, but is not limited thereto, and it may be prescribed in various ways depending on factors such as the formulation method, administration method, the patient's age, weight, sex, pathological condition, food, administration time, administration route, excretion rate, and reaction sensitivity.

The pharmaceutical composition may be prepared in a unit dosage form or it may be prepared by placing it into a multi-dose container by formulating it using a pharmaceutically acceptable carrier and/or excipient according to a method that may be easily implemented by a person skilled in the art to which the present invention pertains.

The formulation may be in the form of a solution, suspension or emulsion in oil or an aqueous medium, or it may be in the form of an extract, powder, granules, tablets or capsules, and may further contain a dispersant and/or stabilizer.

Another aspect provides a method of preventing or treating a neurodegenerative disease, including administering a therapeutically effective amount of the pharmaceutical composition including a peptide consisting of amino acid sequence of any one of SEQ ID NO: 1 to SEQ ID NO: 8 and SEQ ID NO: 20 to SEQ ID NO: 23 as an active ingredient.

Among the terms or elements mentioned in the description of the peptide or pharmaceutical composition, those that are the same as those already mentioned are as described above.

### [Advantageous Effects]

A peptide according to one aspect can inhibit the expression of amyloid precursor protein and increase the expression of ADAM 10, ADAM 17, and IDE, which are genes associated with the degradation of amyloid precursor protein.

In addition, a peptide according to one aspect can alleviate or improve a neuroinflammatory response by increasing the production of anti-inflammatory cytokines and induce neuron regeneration such as neurite growth.

Therefore, a peptide according to one aspect can be used as an active ingredient in a pharmaceutical composition for preventing or treating a neurodegenerative disease.

### [Description of Drawings]

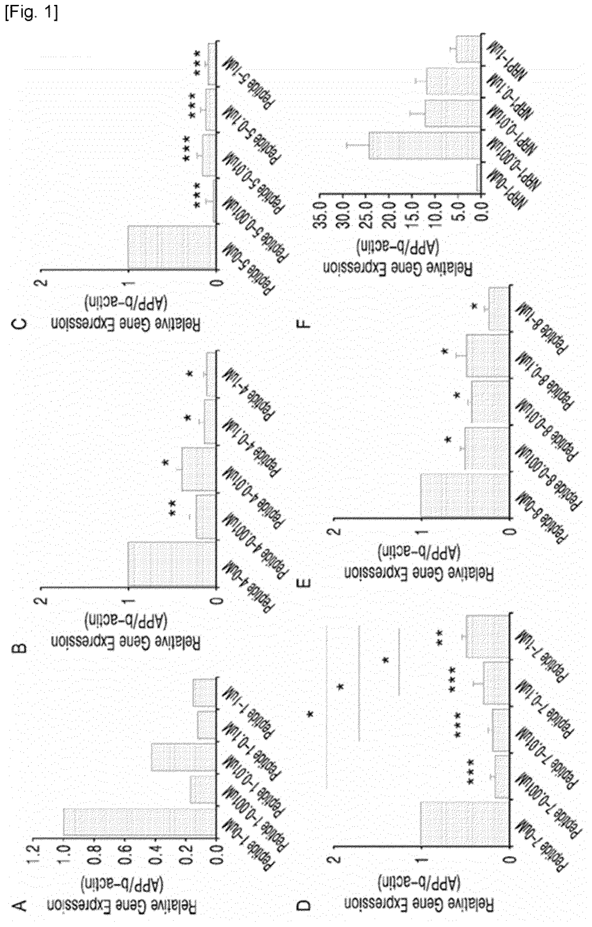

FIG. 1 shows the results confirming the inhibition of the expression of amyloid precursor protein (APP) after adding a peptide according to one embodiment to a human neural cell line, and FIG. 1A shows the results of adding Peptide-1, FIG. 1B shows the results of adding Peptide-4, FIG. 1C shows the results of adding Peptide-5, FIG. 1D shows the results of adding Peptide-7, FIG. 1E shows the results of adding Peptide-8, and FIG. 1F shows the results of adding a comparative group peptide.

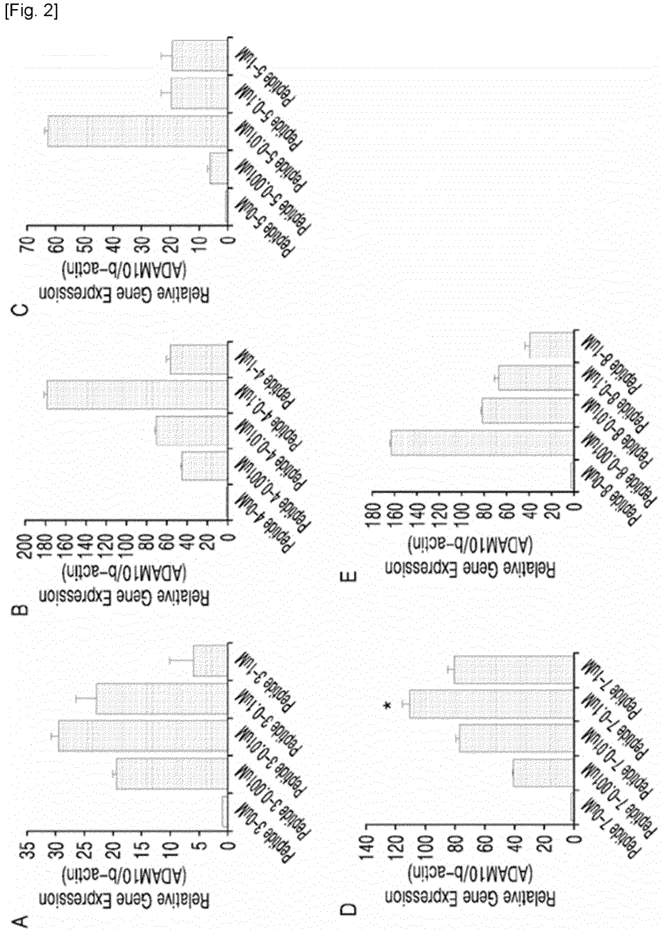

FIG. 2 shows the results confirming the increase in expression of ADAM 10 after adding a peptide according to one embodiment to a human neural cell line, and FIG. 2A shows the results of adding Peptide-3, FIG. 2B shows the results of adding Peptide-4, FIG. 2C shows the results of adding Peptide-5, FIG. 2D shows the results of adding Peptide-7, and FIG. 2E shows the results of adding Peptide-8.

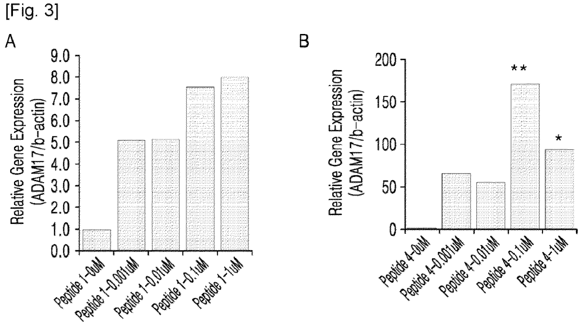

FIG. 3 shows the results confirming the increase in expression of ADAM 17 after adding a peptide according to one embodiment to a human neural cell line, and FIG. 3A shows the results of adding Peptide-1 and FIG. 3B shows the results of adding Peptide-4.

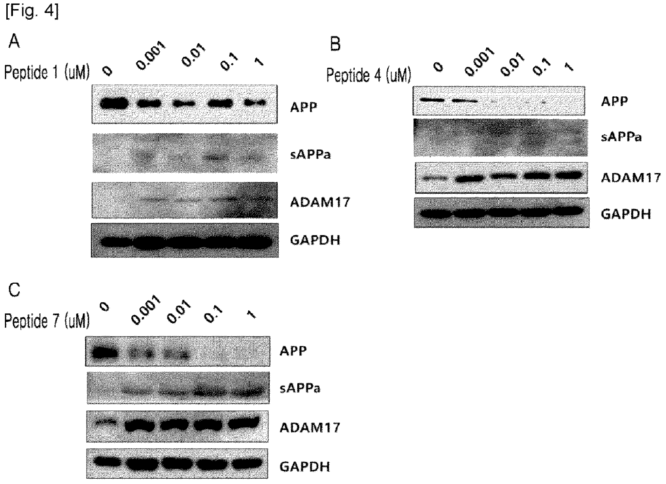

FIG. 4 shows the results confirming the increase in expression of sAPPa and ADAM 17 after adding a peptide according to one embodiment to a human neural cell line, and FIG. 4A shows the results of adding Peptide-1, FIG. 4B shows the results of adding Peptide-4, and FIG. 4C shows the results of adding Peptide-7.

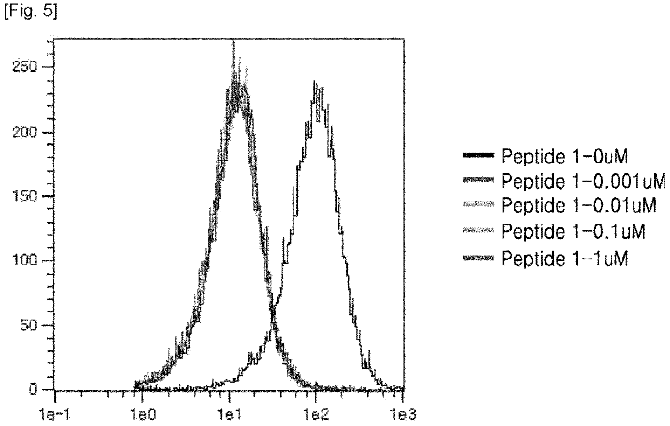

FIG. 5 shows the results of analyzing the distribution of APP on a nerve cell membrane surface by adding a peptide according to one embodiment using flow cytometric analysis.

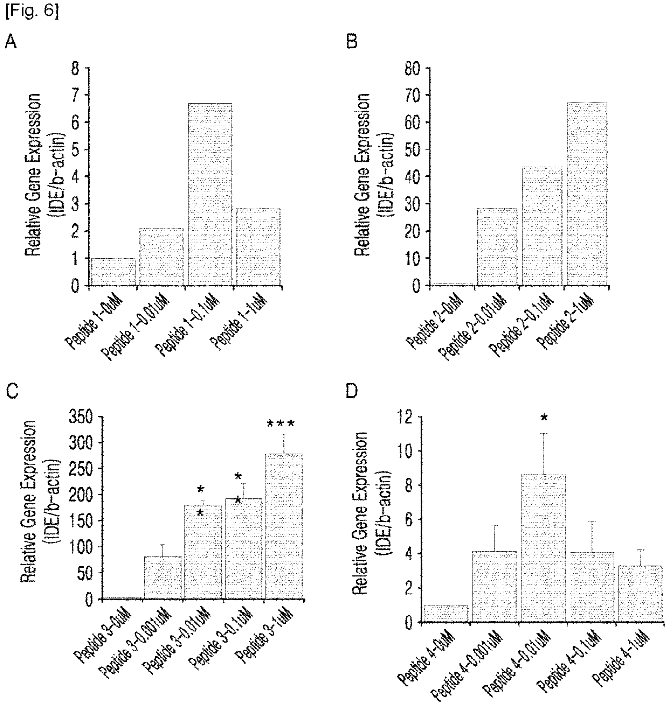

FIG. 6 shows the results confirming the increase in expression of IDE after adding a peptide according to one embodiment to a human neural cell line, and FIG. 6A shows the results of adding Peptide-1, FIG. 6B shows the results of adding Peptide-2, and FIG. 6C shows the results of adding Peptide-3, and FIG. 6D shows the results of adding Peptide-4.

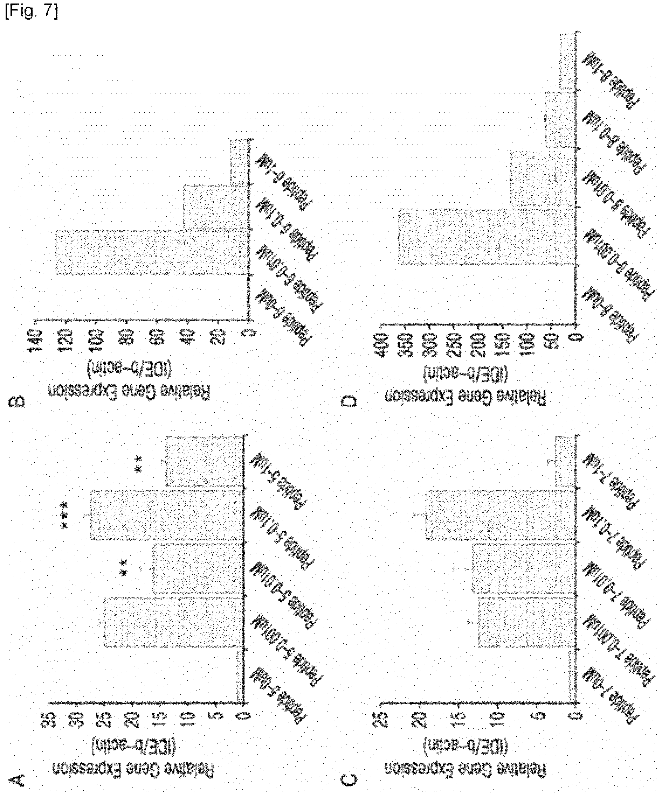

FIG. 7 shows the results confirming the increase in expression of IDE after adding a peptide according to one embodiment to a human neural cell line, and FIG. 7A shows the results of adding Peptide-5, FIG. 7B shows the results of adding Peptide-6, FIG. 7C shows the results of adding Peptide-7, and FIG. 7D shows the results of adding Peptide-8.

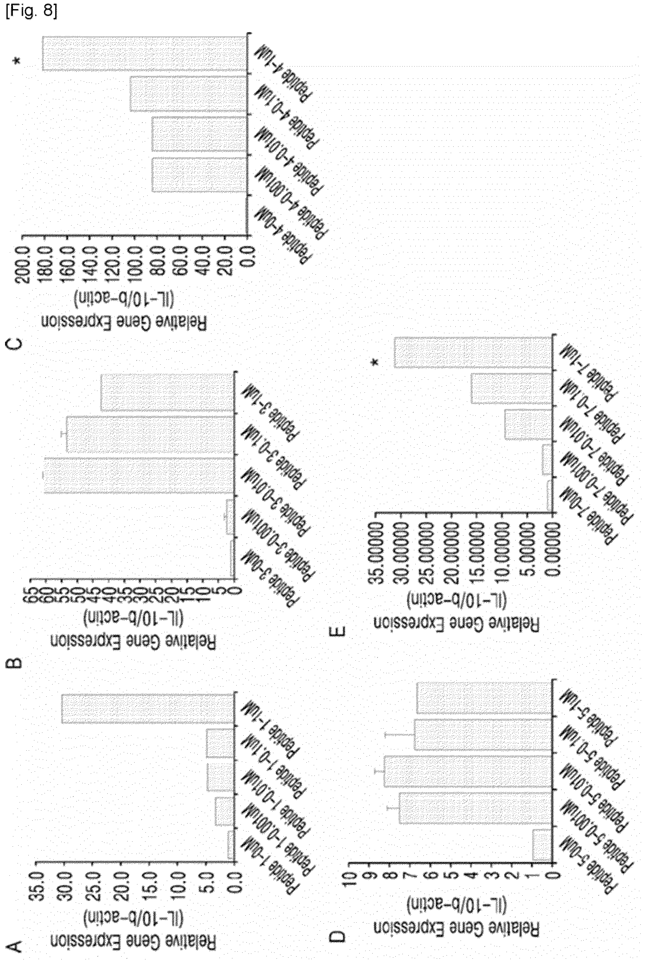

FIG. 8 shows the results confirming the increase in expression of anti-inflammatory cytokines due to the addition of a peptide according to one embodiment, and FIG. 8A shows the results of adding Peptide-1, FIG. 8B shows the results of adding Peptide-3, FIG. 8C shows the results of adding Peptide-4, FIG. 8D shows the results of adding Peptide-5, and FIG. 8E shows the results of adding Peptide-7.

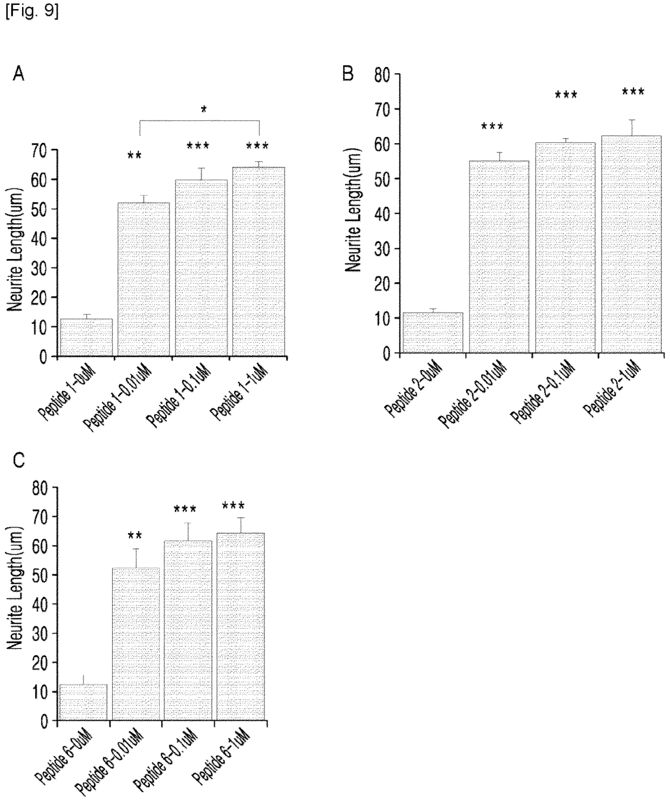

FIG. 9 shows the results confirming the effect of promoting neurite growth after adding a peptide according to one embodiment to a human nerve cell line, and FIG. 9A shows the results of adding Peptide-1, FIG. 9B shows the results of adding Peptide-2, and FIG. 9C shows the results of adding Peptide-6.

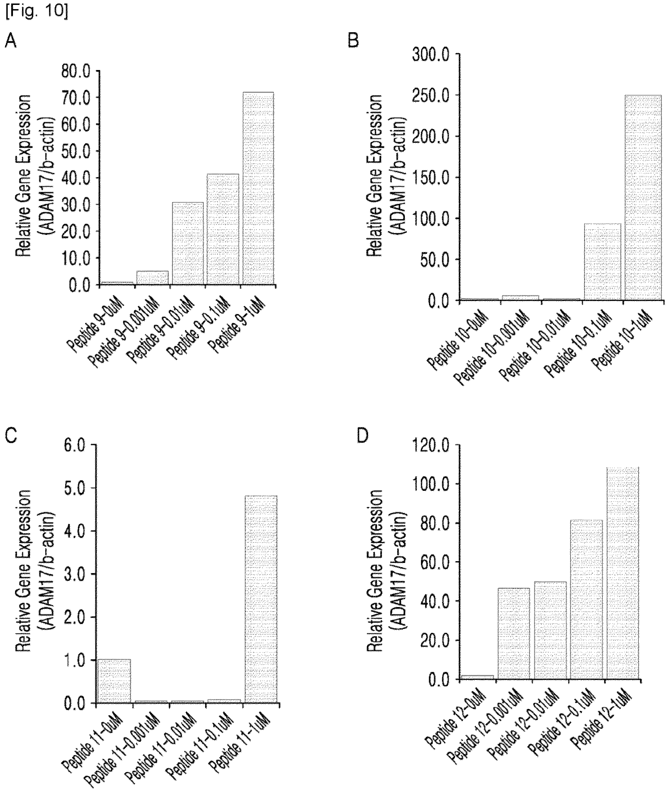

FIG. 10 shows the results confirming the increase in expression of ADAM 17 after adding a peptide according to one embodiment to a human neural cell line, and FIG. 10A shows the results of adding Peptide-9, FIG. 10B shows the results of adding Peptide-10, FIG. 10C shows the results of adding Peptide-11, and FIG. 10D shows the results of adding Peptide-12.

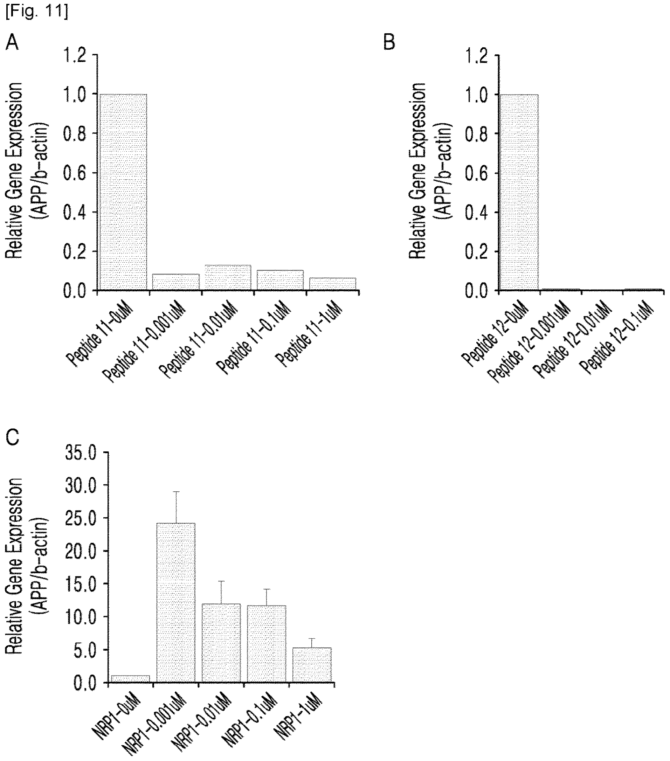

FIG. 11 shows the results confirming the inhibition of the expression of amyloid precursor protein (APP) after adding a peptide according to one embodiment to a human neural cell line, and FIG. 11A shows the results of adding Peptide-11, FIG. 11B shows the results of adding Peptide-12, and FIG. 11C shows the results of adding a comparative group peptide.

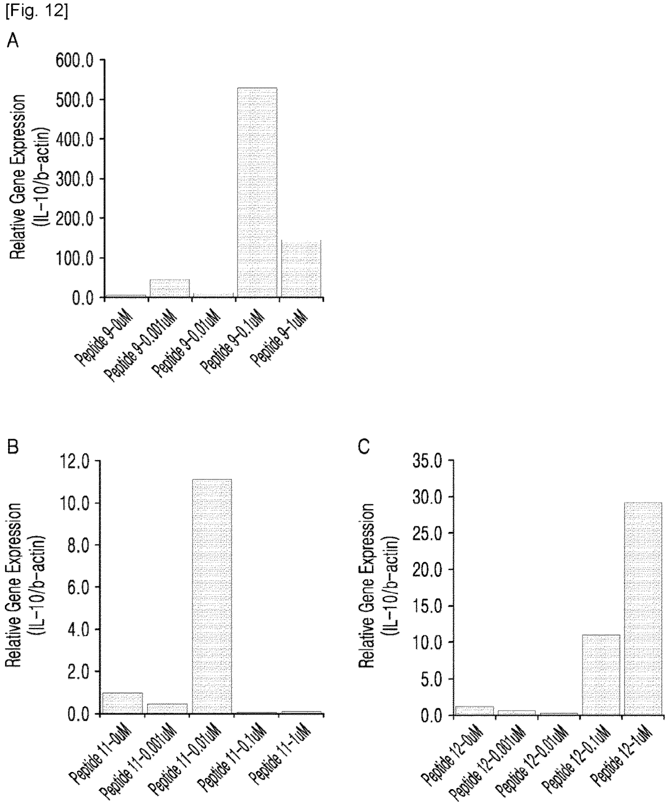

FIG. 12 shows the results of confirming the increase in expression of anti-inflammatory cytokines by the addition of a peptide according one embodiment, and FIG. 12A shows the results of adding Peptide-9, FIG. 12B shows the results of adding Peptide-11, and FIG. 12C shows the results of adding Peptide-12.

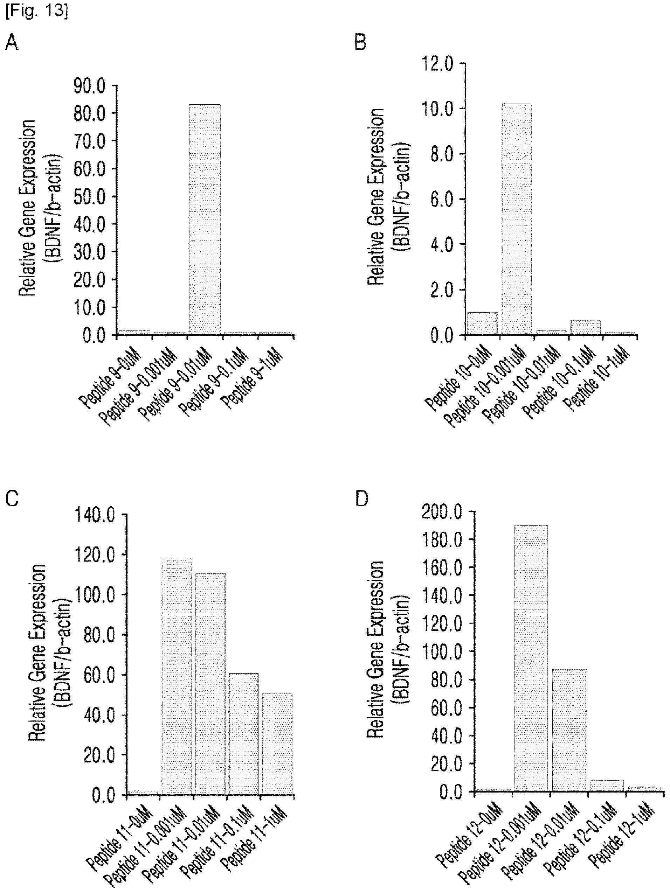

FIG. 13 shows the results confirming the increase in expression of brain-derived nerve growth factor by the addition of a peptide according to one embodiment, and FIG. 13A shows the results of adding Peptide-9, FIG. 13B shows the results of adding Peptide-10, FIG. 13C shows the results of adding Peptide-11, and FIG. 13D shows the results of adding Peptide-12.

### [Modes of the Invention]

Hereinafter, the present invention will be described in more detail through examples. However, these examples are for illustrative purposes only, and the scope of the present invention is not limited to these examples.

### I. Peptide-1 to Peptide-8

### Example 1: Synthesis of peptides

Peptides having the amino acid sequences of SEQ ID NO: 1 to SEQ ID NO: 8 shown in Table 1 below were synthesized using an automatic peptide synthesizer (Milligen 9050, Millipore Corporation, USA), and C18 reversed-phase high-performance liquid chromatography (HPLC) (Waters Associates Corporation, USA) was used to purify these synthesized peptides. The column used was ACQUITY UPLC BEH300 C18 (2.1 mm X 100 mm, 1.7 µm, Waters Co., USA).

**[Table 1]**

| Name | Amino Acid Sequence (N'→C') | SEQ ID NO |
|---|---|---|
| Peptide-1 | LKLKALAALIKKIL | 1 |
| Peptide-2 | IKLLSIVAWAKKKI | 2 |
| Peptide-3 | INLKALAAKIKII, | 3 |
| Peptide-4 | INIKVLIAKKII, | 4 |
| Peptide-5 | LNIRGLGAKKKI | 5 |
| Peptide-6 | LNLRGLAKKKL | 6 |
| Peptide-7 | KALAALAKKII, | 7 |
| Peptide-8 | KALAALAKK | 8 |

### Example 2: Confirmation of effect of inhibiting amyloid precursor protein expression

In the present example, the effect of inhibiting the expression of APP by peptide treatment was confirmed. To this end, the prepared peptides were added at a concentration of 0.001, 0.01, 0.1, or 1 mM to SH-SY5Y, which is a human neural cell line obtained by isolating cells from the bone marrow of a human having neuroblastoma and proliferating them, and then the cells were cultured for 24 hours. Afterward, total RNA was extracted from the culture, cDNA was synthesized, and real-time quantitative polymerase chain reaction (qPCR) was performed using the APP primers shown in Table 2 below to measure the expression level of amyloid precursor protein (APP). Meanwhile, a group to which no peptide was added was used as a control group, and a group to which a random peptide was added (NRP1: AFMVDNEAIYDICR (SEQ ID NO: 9)) was used as a comparative group.

**[Table 2]**

| Name | Sequence (5' → 3') | SEQ ID NO |
|---|---|---|
| APP Forward | CCGAGGGGTAGAGTTTGTGT | 10 |
| APP Reverse | ACCTCAGCCACTTCTTCCTC | 11 |

As a result, as shown in FIG. 1, by the addition of a peptide (Peptide-1, Peptide-4, Peptide-5, Peptide-7, or Peptide-8) according to one embodiment, the expression of APP, which is a main causative protein of a neurodegenerative disease including AD, was inhibited. In particular, since the expression level was about 80% to 90% lower than that of the control group, the effect of inhibiting the expression of APP was very significant.

### Example 3: Confirmation of effect of increasing expression of genes associated with degradation of amyloid precursor protein expression

### 3-1. Expression of ADAM 10 (A Disintegrin And Metalloproteinase-containing protein 10) gene

ADAM 10 is an alpha-secretase that cleaves APP into a non-toxic fragment and inhibits the production of beta-amyloid (β-amyloid, Ab 1-42, 1-40). Therefore, the increased expression of ADAM 10 may be used as an indicator of a therapeutic effect on a neurodegenerative disease. Specifically, the prepared peptides were added to SH-SY5Y, a human neural cell line, at a concentration of 0.001, 0.01, 0.1, or 1 mM, and the cells were cultured at 37 °C and 5% CO₂ for 24 hours. Afterwards, total RNA was extracted from the culture, cDNA was synthesized, and real-time qPCR was performed using the ADAM 10 primers shown in Table 3 below to measure the expression level of ADAM 10. Meanwhile, a group to which no peptide was added was used as a control group.

As a result, as shown in FIG. 2, the expression of ADAM 10 was increased by the addition of a peptide (Peptide-3, Peptide-4, Peptide-5, Peptide-7, or Peptide-8) according to one embodiment.

**[Table 3]**

| Name | Sequence (5' → 3') | SEQ ID NO |
|---|---|---|
| ADAM 10 Forward | ACCACAGACTTCTCCGGAATC | 12 |
| ADAM 10 Reverse | GGTCTGTGAAGACATAGGCCA | 13 |

### 3-2. Expression of ADAM 17 (A Disintegrin And Metalloproteinase-containing protein 17) gene

ADAM 17 is an enzyme that removes APP from the cell membrane surface by cleaving it into a non-toxic fragment, sAPPa. Therefore, the increased expression of ADAM 17 may be used as an indicator of a therapeutic effect on a neurodegenerative disease. Specifically, the prepared peptides were added to SH-SY5Y, a neural cell line, at a concentration of 0.001, 0.01, 0.1, or 1 mM, and cultured at 37 °C and 5% CO₂ for 24 hours. Afterward, total RNA was extracted from the culture, cDNA was synthesized, and real-time qPCR was performed using the ADAM 17 primers shown in Table 4 below to measure the expression level of ADAM 10. In addition, the prepared peptides were added to SH-SY5Y, a human neural cell line, at a concentration of 0.001, 0.01, 0.1, or 1 mM, and cultured at 37 °C and 5% CO₂ for 24 hours. Afterward, the expression of ADAM 17 protein from the culture and the expression of sAPPa, which was cleaved from APP by ADAM 17 and ADAM 10 and released, were confirmed through Western blotting using antibodies against each protein. In addition, the distribution of APP on the surface of a nerve cell membrane was analyzed using flow cytometric analysis. Meanwhile, a group to which no peptide was added was used as a control group.

**[Table 4]**

| Name | Sequence (5' → 3') | SEQ ID NO |
|---|---|---|
| ADAM 17 Forward | TCGCTTCTACAGCATGGGC | 14 |
| ADAM 17 Reverse | TGAGAATGCGAATCTGCTCT | 15 |

As a result, as shown in FIGS. 3 and 4, the expression of sAPPa and ADAM 17 was increased by the addition of a peptide (Peptide-1, Peptide-4, and Peptide-7) according to one embodiment, and as shown in FIG. 5, it can be seen that the expression change contributed to reducing the level of APP present on the surface of the nerve cell membrane.

### 3-3. Expression of IDE (Insulin degrading enzyme) gene

IDE is an enzyme that degrades senile plaques produced and aggregated by beta-amyloid (Ab1-42, 1-40). Therefore, the increased expression of IDE may be used as an indicator of a therapeutic effect of a neurodegenerative disease. Specifically, the prepared peptides were added to SH-SY5Y, a human neural cell line, at a concentration of 0.01, 0.1, or 1 mM, and the cells were cultured at 37 °C and 5% CO₂ for 24 hours. Afterward, total RNA was extracted from the culture, cDNA was synthesized, and real-time qPCR was performed using the IDE primers in Table 5 below to measure the expression level of IDE. Meanwhile, a group to which no peptide was added was used as a control group.

**[Table 5]**

| Name | Sequence (5' → 3') | SEQ ID NO |
|---|---|---|
| IDE Forward | AAAGAGGCGAGACCATACCC | 16 |
| IDE Reverse | GCAAGGGGTCCACATAAGCA | 17 |

As a result, as shown in FIGS. 6 and 7, the expression of IDE was increased by the addition of a peptide (Peptide-1, Peptide-2, Peptide-3, Peptide-4, Peptide-5, Peptide-6, Peptide-7, and Peptide-8) according to one embodiment.

Summarizing the above experimental results, a peptide according to one embodiment may increase the expression of ADAM 10 and ADAM 17, which cleave APP on a cell membrane surface to release it in the form of sAPPa, a non-toxic fragment, and increase the expression of IDE, which degrades senile plaques in brain tissue resulting from pathological factors, thereby preventing the onset of a neurodegenerative disease or improving related symptoms.

### Example 4: Confirmation of effect of increasing expression of anti-inflammatory cytokines

In the present example, to confirm the effect inhibiting a neuroinflammatory response by peptide treatment, which is one of the main causes of a neurodegenerative disease, the effect of increasing the expression of IL-10, one of the anti-inflammatory cytokines, was confirmed. Specifically, the prepared peptides were added to SH-SY5Y, a human neural cell line, at a concentration of 0.001, 0.01, 0.1, or 1 mM, and the cells were cultured at 37 °C and 5% CO₂ for 24 hours. Afterward, total RNA was extracted from the culture, cDNA was synthesized, and real-time qPCR was performed using the IL-10 primers in Table 6 below to measure the expression level of IL-10. Meanwhile, a group to which no peptide was added was used as a control group.

**[Table 6]**

| Name | Sequence (5' → 3') | SEQ ID NO |
|---|---|---|
| IL-10 Forward | CCCCAAGCTGAGAACCA | 18 |
| IL-10 Reverse | TCTCAAGGGGCTGGGTCAGC | 19 |

As a result, as shown in FIG. 8, the expression of II,-10 was increased by the addition of a peptide (Peptide-1, Peptide-3, Peptide-4, Peptide-5, or Peptide-7) according to one embodiment.

### Example 5: Confirmation of neurite growth promoting effect

In the present example, to confirm the regeneration of neurons by treatment with the peptides, the growth of neurites was confirmed. Specifically, the prepared peptides were added to SH-SY5Y, a human neural cell line, at a concentration of 0.01, 0.1, or 1 mM, and the cells were cultured for 24 hours. Afterward, the grown neurites were confirmed through a microscope, and their length was measured and evaluated.

As a result, as shown in FIG. 9, the length of the neurites was increased by the addition of a peptide (Peptide-1, Peptide-2, or Peptide-6) according to one embodiment.

Through the above results, it can be seen that a peptide according to one embodiment may contribute to suppressing the onset and progression of a neurodegenerative disease including AD, Parkinson's disease, Huntington's disease, and multiple sclerosis.

### II. Peptide-9 to Peptide-12

### Example 1: Synthesis of peptides

Peptides having the amino acid sequences of SEQ ID NO: 20 to SEQ ID NO: 23 shown in Table 7 below were synthesized using an automatic peptide synthesizer (Milligen 9050, Millipore Corporation, USA), and C18 reversed-phase (HPLC) (Waters Associates Corporation, USA) was used to purify these synthesized peptides. The column used was ACQUITY UPLC BEH300 C18 (2.1 mm X 100 mm, 1.7 µm, Waters Co., USA).

**[Table 7]**

| Name | Amino Acid Sequence (N'→C') | SEQ ID NO |
|---|---|---|
| Peptide-9 | INLLKIAGKIINIL | 20 |
| Peptide-10 | INLLKSEGKIIKSL | 21 |
| Peptide-11 | GRPPGFSLAR | 22 |
| Peptide-12 | GRILGFSPFR | 23 |

### Example 2: Confirmation of effect of increasing expression of genes associated with degradation of amyloid precursor protein

The prepared peptides were added to SH-SY5Y, a neural cell line, at a concentration of 0.001, 0.01, 0.1, or 1 mM, and the cells were cultured at 37 °C and 5% CO₂ for 24 hours. Afterward, total RNA was extracted from the culture, cDNA was synthesized, and real-time qPCR was performed using the ADAM 17 primers in Table 4 above to measure the expression level of ADAM 17. Meanwhile, a group to which no peptide was added was used as a control group.

As a result, as shown in FIG. 10, the expression of ADAM 17 was increased by the addition of a peptide (Peptide-9, Peptide-10, Peptide-11, or Peptide-12) according to one embodiment, and this tendency was dependent on the concentration.

Summarizing the above experimental results, a peptide according to one embodiment may increase the expression of ADAM 17, which cleaves APP on a cell membrane surface to release it in the form of sAPPa, a non-toxic fragment, thereby preventing the onset of a neurodegenerative disease or improving related symptoms.

### Example 3: Confirmation of effect of inhibiting amyloid precursor protein expression

In the present example, the effect of inhibiting the expression of **amyloid precursor protein** by peptide treatment was confirmed. To this end, the prepared peptides were added at a concentration of 0.001, 0.01, 0.1, or 1 mM to SH-SY5Y, which is a human neural cell line obtained by isolating cells from the bone marrow of a human having neuroblastoma and proliferating them, and then the cells were cultured for 24 hours. Afterward, total RNA was extracted from the culture, cDNA was synthesized, and real-time qPCR was performed using the APP primers shown in Table 2 above to measure the expression level of APP. Meanwhile, a group to which no peptide was added was used as a control group, and a group to which a random peptide was added (NRP1: AFMVDNEAIYDICR (SEQ ID NO: 9)) was used as a comparative group.

As a result, as shown in FIG. 11, the expression of APP, a major causative protein of a neurodegenerative disease including AD, was inhibited by the addition of a peptide (Peptide-11 or Peptide-12) according to one embodiment. In particular, since the expression level was about 80% to 90% lower than that of the control group, the effect of inhibiting the expression of APP was very significant.

### Example 4: Confirmation of effect of increasing expression of anti-inflammatory cytokines

In the present example to confirm the effect of inhibiting a neuroinflammatory response, which is one of the main causes of a neurodegenerative disease, by peptide treatment, the effect of increasing the expression of IL-10, one of the anti-inflammatory cytokines, was attempted. Specifically, the prepared peptide was added to SH-SY5Y, a human neural cell line, at a concentration of 0.001, 0.01, 0.1, or 1 mM, and the cells were cultured at 37 °C and 5% CO₂ for 24 hours. Afterward, total RNA was extracted from the culture, cDNA was synthesized, and real-time qPCR was performed using the IL-10 primers in Table 6 above to measure the expression level of IL-10. Meanwhile, a group to which no peptide was added was used as a control group.

As a result, as shown in FIG. 12, the expression of IL-10 was increased by the addition of a peptide (Peptide-9, Peptide-11, or Peptide-12) according to one embodiment. In particular, this anti-inflammatory effect was more significant in the group to which Peptide-9 was added.

Summarizing the above experimental results, a peptide according to one embodiment may inhibit the degradation of APP and at the same time, induce an anti-inflammatory response at the site of a lesion.

### Example 5. Confirmation of effect of increasing expression of brain nerve growth factor

In the present example, to confirm the effect of improving or treating a degenerative neurological disease through the regeneration of the nervous system by peptide treatment, the effect of increasing the expression of Brain Derived Neurotrophic Factor (BDNF) was examined. Specifically, the prepared peptides were added to SH-SY5Y, a human neural cell line, at a concentration of 0.001, 0.01, 0.1, or 1 mM, and the cells were cultured at 37 °C and 5% CO₂ for 24 hours. Afterward, total RNA was extracted from the culture, cDNA was synthesized, and real-time qPCR was performed using the BDNF primers shown in Table 8 below to measure the expression level of BDNF. Meanwhile, a group to which no peptide was added was used as a control group.

**[Table 8]**

| Name | Sequence (5' → 3') | SEQ ID NO |
|---|---|---|
| BDNF Forward | GGCGGCAGACAAAAAGACTG | 24 |
| BDNF Reverse | CACTGGGAGTTCCAATGCCT | 25 |

As a result, as shown in FIG. 13, the expression of BDNF was increased by the addition of a peptide (Peptide-9, Peptide-10, Peptide-11, or Peptide-12) according to one embodiment. In particular, this effect of increasing the expression was more significant in the group to which Peptide-11 or Peptide-12 was added.

Through the above results, it can be seen that a peptide according to one embodiment may contribute to suppressing the onset and progression of a neurodegenerative disease including AD, Parkinson's disease, Huntington's disease, and multiple sclerosis.

The description of the present invention described above is for illustrative purposes, and those skilled in the art will understand that the present invention may be easily modified into other specific forms without changing the technical idea or essential features of the present invention. Therefore, the embodiments described above should be understood in all respects as illustrative and not restrictive.

## Claims

1. A peptide consisting of amino acid sequence of any one of SEQ ID NO: 1 to SEQ ID NO: 8 and SEQ ID NO: 20 to SEQ ID NO: 23.

2. The peptide of claim 1, wherein an N-terminus of the peptide is coupled to a protecting group of any one selected from the group consisting of an acetyl group, a fluoreonylmethoxycarbonyl group, a formyl group, a palmitoyl group, a myristyl group, a stearyl group, and polyethylene glycol (PEG).

3. The peptide of claim 1, wherein a C-terminus of the peptide is coupled to a protecting group of any one selected from the group consisting of an amino group (-NH₂) and azide (-NHNH₂).

4. The peptide of claim 1, wherein the peptide exhibits any one or more selected from the following characteristics:
(a) inhibition of amyloid precursor protein (APP) expression;
(b) increased expression of a disintegrin and metalloproteinase-containing protein 10 (ADAM 10) protein or a disintegrin and metalloproteinase-containing protein 17 (ADAM 17) protein;
(c) increased expression of insulin degrading enzyme (IDE) protein;
(d) increased expression of anti-inflammatory cytokines;
(e) promotion of neurite growth; and
(f) increased expression of brain derived neurotrophic factor (BDNF).

5. A pharmaceutical composition for preventing or treating a neurodegenerative disease, comprising the peptide of any one of claims 1 to 4 as an active ingredient.

6. The pharmaceutical composition of claim 5, wherein the neurodegenerative disease is selected from the group consisting of Alzheimer's disease, Parkinson's disease, Huntington's disease, multiple sclerosis, mild cognitive impairment, cerebral amyloid angiopathy, amyloid stroke, systematic amyloidosis, amyloidosis-Dutch type, Niemann-Pick disease, senile dementia, amyotrophic lateral sclerosis, spinocerebellar atrophy, Tourette's syndrome, Friedreich's ataxia, Machado-Joseph's disease, Lewy body dementia, dystonia, progressive supranuclear palsy, frontotemporal dementia, peripheral neuropathy, and polyneuropathy.

7. The pharmaceutical composition of claim 5, wherein the neurodegenerative disease is Alzheimer's disease.
